Europäisches Patentamt

European Patent Office

Office européen des brevets

(11) Numéro de publication: **0 228 309**
**A1**

(19)

(12) **DEMANDE DE BREVET EUROPEEN**

(21) Numéro de dépôt: 86402352.8

(22) Date de dépôt: 20.10.86

(51) Int. Cl.4: **G 01 T 1/29**
G 01 T 1/161

(30) Priorité: 25.10.85 FR 8515887

(43) Date de publication de la demande:
08.07.87 Bulletin 87/28

(84) Etats contractants désignés: **DE GB SE**

(71) Demandeur: **COMMISSARIAT A L'ENERGIE ATOMIQUE**
31/33, rue de la Fédération
**F-75015 Paris (FR)**

(72) Inventeur: **Lecomte, Jean-Luc**
**14 Allée F. Villon**
**F-388130 Echirolles (FR)**

**Martin, Michel**
**La Côte Herbeys**
**F-38320 Eybens (FR)**

**Sauvage, Francis**
**34, rue Victor Hugo**
**F-38430 Moirans (FR)**

**Tournier, Edmond**
**2 Place Léon Martin**
**F-38000 Grenoble (FR)**

(74) Mandataire: **Mongrédien, André et al**
**c/o BREVATOME 25, rue de Ponthieu**
**F-75008 Paris (FR)**

(54) **Dispositif d'étalonnage pour appareils de médecine nucléaire et son procédé de mise en oeuvre.**

(57) Ce dispositif comprend une source radioactive liquide (1) servant en dehors de la présence d'un patient (30), à l'étalonnage des détecteurs de rayonnements ionisants de l'appareil, logée dans un conteneur (3), pouvant contenir ladite source, caractérisé en ce qu'il comprend un magasin de stockage (5) communiquant avec le conteneur par au moins une canalisation (9) et des moyens pour transférer la source liquide dudit magasin de stockage au conteneur et inversement, le conteneur étant fixe par rapport audit appareil et le magasin ayant un volume au moins égal à celui du conteneur.

Application à l'étalonnage de détecteurs de rayonnements ionisants utilisés dans des appareils de médecine nucléaire tels que les tomographes à positons, les gamma-caméras, les tomographes gammas.

FIG. 2

## Description

DISPOSITIF D'ÉTALONNAGE POUR APPAREILS DE MÉDECINE NUCLÉAIRE ET SON PROCÉDE DE MISE EN OEUVRE

La présente invention concerne un dispositif d'étalonnage pour appareils de médecine nucléaire et son procédé de mise en oeuvre.

Cette invention s'applique l'étalonnage de détecteurs de rayonnements ionisants, utilisés dans des appareils de médecine nucléaire pour l'observation d'un patient préalablement rendu radioactif. Ces appareils de médecine nucléaire sont par exemple des tomographes à positons, des gamma-caméras, des tomographes gammas.

De façon connue, pour étalonner les détecteurs de rayonnements ionisants d'un appareil de médecine nucléaire, on utilise une source radioactive liquide maintenue dans un conteneur. Ce conteneur est placé dans la zone de détection de ces détecteurs, avant chaque observation d'un patient.

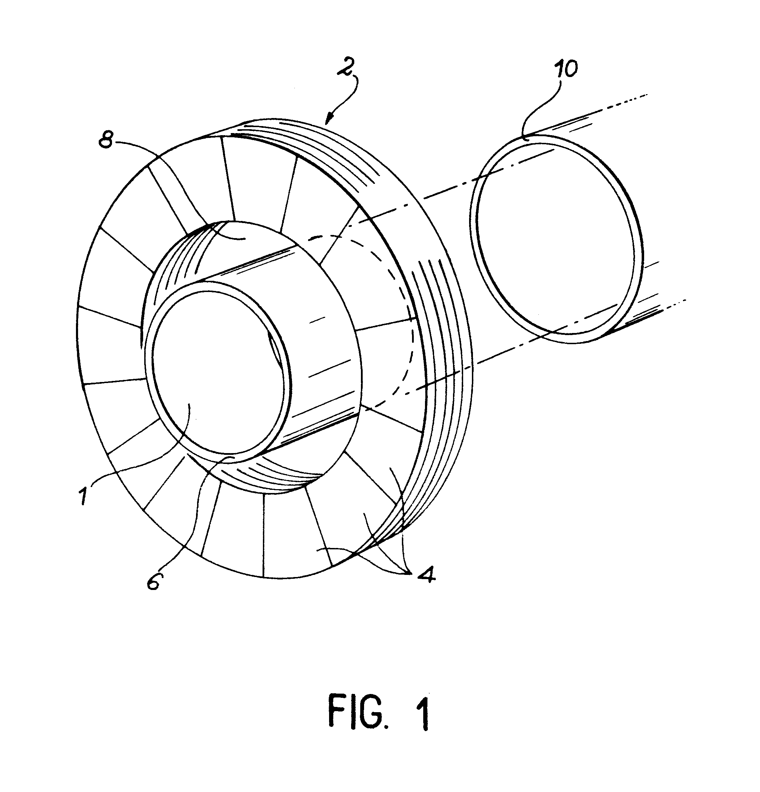

La figure 1 représente schématiquement un appareil de médecine nucléaire 2, à l'intérieur duquel est placé un conteneur 6 de type connu, contenant une source radioactive liquide 1.

Cet appareil 2 comprend un ensemble de détecteurs 4 disposés, par exemple dans le cas d'un appareil de tomographie, de façon à réaliser un cylindre ou une portion de cylindre. La zone interne audit cylindre, correspond à la zone de détection 8 de l'appareil.

L'ensemble de ces détecteurs 4 est généralement mobile afin de pouvoir observer le patient dans toutes les directions de l'espace, lorsque celui-ci est placé au centre de la zone de détection 8.

Le conteneur 6 a une forme adaptée à l'ensemble des détecteurs 4 ; il est donc cylindrique dans le cas d'un appareil de tomographie et de longueur généralement supérieure à celui-ci.

Le conteneur 6 est constitué comme représenté figure 1 par une chambre annulaire contenant la source radioactive liquide 1, ou bien par un enroulement de spires remplies de ladite source 1.

Lors d'une opération d'étalonnage, ce conteneur 6 est placé dans la zone de détection 8 de façon à ce qu'il soit centré par rapport à l'ensemble des détecteurs 4.

Après chaque étalonnage, le conteneur 6 est retiré de la zone de détection 8 pour ne pas perturber les mesures effectuées sur un patient placé au centre de la zone de détection 8. Ce conteneur 6 est alors placé dans une enceinte 10 blindée, généralement en plomb, pour protéger l'environnement des rayonnements ionisants émis par la source 1 dans le conteneur 6.

De façon connue, le conteneur 6 est placé alternativement dans la zone de détection 8 et dans l'enceinte 10 soit manuellement par un opérateur, soit par des moyens de commande automatique de déplacement (non représentés).

La méthode manuelle d'introduction du conteneur 6 dans la zone de détection 8 et son rangement dans l'enceinte 10 permet de ne pas encombrer la zone de détection par des dispositifs de commande. En revanche, cette méthode ne préserve pas le personnel de rayonnements ionisants.

La méthode de commande automatique nécessite de disposer l'enceinte 10 au moins en partie dans la zone de détection. Des commandes automatiques de déplacement permettent alors de placer le conteneur 6 soit au centre de la zone de détection 8, soit dans l'enceinte 10. Cette méthode permet donc d'éviter l'exposition du personnel mais nécessite de placer les moyens de commande automatique de déplacement et l'enceinte au moins en partie dans la zone de détection, ce qui conduit à un encombrement important de cette zone.

L'invention a pour but de remédier à ces inconvénients en réalisant un dispositif d'étalonnage comprenant un conteneur fixe par rapport à la zone de détection d'un appareil de médecine nucléaire, ce conteneur contenant la source radioactive liquide uniquement pendant les opérations d'étalonnage de cet appareil ; le dispositif de l'invention permet à la fois de ne pas exposer le personnel à des rayonnements ionisants et de ne pas encombrer la zone de détection par des dispositifs de commande.

De façon plus précise, l'invention a pour objet un dispositif d'étalonnage pour appareil de médecine nucléaire servant à l'observation d'un patient préalablement rendu radioactif, comprenant une source radioactive liquide, servant à l'étalonnage des détecteurs de rayonnements ionisants de l'appareil, logée dans un conteneur pouvant contenir ladite source, caractérisé en ce qu'il comprend un magasin de stockage communiquant avec le conteneur par au moins une canalisation et des moyens pour transférer la source radioactive liquide dudit magasin de stockage au conteneur et inversement, le conteneur étant fixe par rapport audit appareil et le magasin de stockage ayant un volume au moins égal à celui du conteneur.

Le magasin de stockage est disposé dans une zone permettant de ne pas encombrer la zone de détection. Ce magasin comprend des moyens pour arrêter des rayonnements ionisants constitués soit par les parois mêmes du magasin, soit par des protections telles que des plaques en plomb entourant ledit magasin. Par contre, le conteneur est réalisé en un matériau laissant passer ces rayonnements, tels que l'acier inoxydable ou l'aluminium.

Selon un mode préféré de réalisation du dispositif d'étalonnage, les moyens pour transférer la source radioactive liquide comprennent une première membrane élastique étanche, disposée dans le conteneur de façon à créer dans ledit conteneur une première et une deuxième chambres à volumes variables, la première chambre étant reliée au magasin de stockage et la deuxième chambre étant reliée à des premiers moyens d'introduction et d'évacuation de gaz sous pression.

Selon un autre mode préféré de réalisation du dispositif d'étalonnage, les moyens pour transférer la source liquide comprennent une deuxième membrane élastique étanche, disposée dans le magasin de stockage de façon à créer dans ledit magasin une première et une deuxième enceintes à volumes variables, la première enceinte étant reliée au conteneur et la deuxième enceinte étant reliée à des deuxièmes moyens d'introduction et d'évacuation de gaz sous pression.

De façon avantageuse, le conteneur est torique et la première membrane est conique, les première et deuxième chambres étant toriques, l'une desdites chambres entourant l'autre.

Dans ce cas, la forme du conteneur est adaptée à la forme d'un ensemble de détecteurs disposés suivant un cylindre comme dans le cas d'un appareil de tomographie.

On entend par forme "torique" du conteneur et des première et deuxième chambres une forme annulaire dont la section est de forme quelconque.

De préférence, les première et deuxième enceintes ont respectivement une forme de soucoupe et sont réunies suivant leur plus grande section par la deuxième membrane.

Selon un autre mode de réalisation du dispositif d'étalonnage, les premiers moyens d'introduction et d'évacuation de gaz comprennent un ballon de réserve de gaz sous pression relié par une canalisation à la deuxième chambre.

Selon un autre mode de réalisation du dispositif d'étalonnage, les deuxièmes moyens d'introduction et d'évacuation de gaz comprennent :
- des moyens d'introduction de gaz comprenant des moyens de stockage de gaz reliés à la deuxième enceinte par une première canalisation comportant des premiers moyens d'ouverture et de fermeture,
- des moyens d'évacuation de gaz par une mise à l'air ambiant de la deuxième enceinte, lesdits moyens comportant des deuxièmes moyens d'ouverture et de fermeture.

De façon avantageuse, ces moyens d'évacuation de gaz par une mise à l'air ambiant de la deuxième enceinte comprennent un vase relié à la première canalisation par une deuxième canalisation, ce vase étant en outre relié à une troisième canalisation ouverte à l'air ambiant, ladite deuxième canalisation comprenant les deuxièmes moyens d'ouverture et de fermeture et des moyens de limitation de pression.

Selon un autre mode de réalisation, le dispositif d'étalonnage comprend des moyens d'évacuation d'air de la première enceinte et de la première chambre. Ces moyens sont reliés de façon avantageuse à la première enceinte, le magasin de stockage étant généralement disposé au-dessus du conteneur ; mais ils peuvent être également reliés à la première chambre.

Selon un autre mode de réalisation, le dispositif d'étalonnage comprend des moyens pour introduire et évacuer la source radioactive liquide notamment pour la renouveler. Ces moyens sont reliés de préférence à la première chambre, le conteneur étant généralement disposé au-dessous du magasin de stockage ; mais ils peuvent être également reliés à la première enceinte.

L'invention a également pour objet un procédé de mise en oeuvre du dispositif d'étalonnage, caractérisé en ce que la source radioactive liquide étant dans la première enceinte, on transfère la source liquide dans la première chambre en introduisant du gaz sous pression dans la deuxième enceinte par les deuxièmes moyens d'introduction et d'évacuation de gaz sous pression.

Selon un autre mode de mise en oeuvre du procédé, la source radioactive liquide étant dans la première chambre, on transfère la source radioactive liquide dans la première enceinte en évacuant le gaz de la deuxième enceinte par les deuxièmes moyens d'introduction et d'évacuation de gaz sous pression.

D'autres caractéristiques et avantages de l'invention ressortiront mieux de la description qui va suivre donnée à titre purement illustratif et non limitatif en référence aux figures 2 à 4 annexées dans lesquelles :

- la figure 2 représente schématiquement un exemple de réalisation d'un dispositif d'étalonnage conforme à l'invention ;
- la figure 3 représente schématiquement le conteneur et le magasin de stockage du dispositif de la figure 2 contenant respectivement du gaz sous pression et la source radioactive liquide ; et
- la figure 4 représente schématiquement le conteneur et le magasin de stockage du dispositif de la figure 2 contenant respectivement la source radioactive liquide et du gaz sous pression.

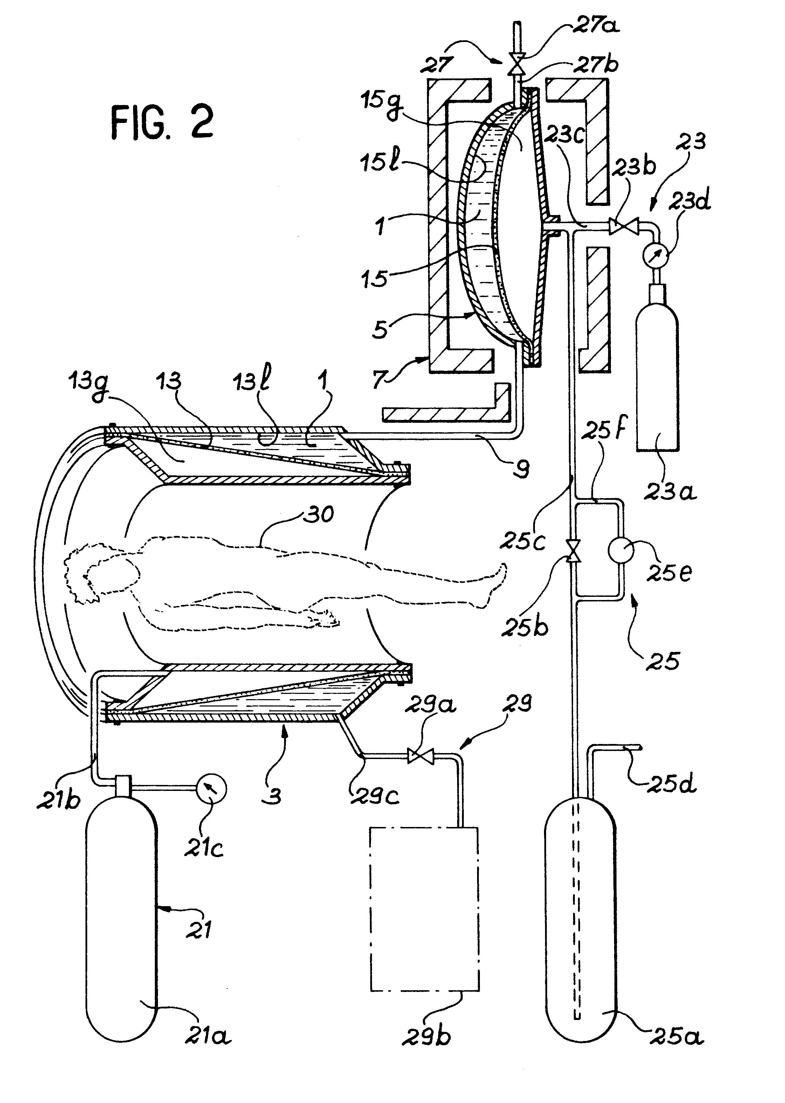

La figure 2 représente un exemple de réalisation d'un dispositif d'étalonnage conforme à l'invention, par exemple pour un appareil de tomographie à positons.

Ce dispositif comprend un conteneur 3 relié par une canalisation 9 à un magasin de stockage 5 et une source radioactive liquide 1 pouvant être transférée du magasin 5 au conteneur 3 et inversement, par des moyens de transfert.

Ces moyens de transfert comprennent une première membrane élastique 13 étanche, disposée dans le conteneur 3 de façon à créer dans celui-ci une première chambre 13l pouvant recevoir la source radioactive liquide 1 et une deuxième chambre 13g pouvant recevoir du gaz sous pression.

Les moyens de transfert comprennent en outre une deuxième membrane élastique 15 étanche, disposée dans le magasin de stockage 5 de façon à créer une première enceinte 15l pouvant recevoir la source 1 et une deuxième enceinte 15g pouvant recevoir du gaz sous pression.

La chambre 13l et l'enceinte 15l sont reliées entre elles par la canalisation 9. Par ailleurs, la chambre 13g est reliée à des moyens 21 d'introduction et d'évacuation de gaz sous pression et l'enceinte 15g est reliée de même à des moyens 23, 25 d'introduction et d'évacuation de gaz sous pression.

Le magasin 5 est généralement à une hauteur supérieure à celle du conteneur 5. Aussi, pour l'évacuation d'air présent dans la chambre 13l et dans l'enceinte 15l, des moyens 27 d'évacuation d'air sont reliés de préférence à l'enceinte 15l au niveau le plus haut de celle-ci.

Ces moyens 27 comprennent par exemple une canalisation 27b reliée à l'enceinte 15l et à l'atmosphère, cette canalisation 27b comprenant des moyens d'ouverture et de fermeture tels qu'une vanne 27a.

De plus, pour introduire la source 1 dans le dispositif de l'invention et pour l'évacuer du dispositif, notamment en cas de rupture de la membrane 13 ou pour le renouvellement de la source 1, des moyens 29 d'introduction et d'évacuation de la source sont reliés de préférence à la chambre 13l à son niveau le plus bas.

Ces moyens 29 comprennent par exemple une canalisation 29c reliée d'une part à la chambre 13l et d'autre part à des moyens 29b pour recevoir la source 1 ou contenant la source 1 à introduire, cette canalisation 29c comprenant des moyens d'ouverture et de fermeture tels qu'une vanne 29a.

Les détecteurs utilisés dans un appareil de tomographie sont répartis suivant un cylindre ou une portion de cylindre. Le conteneur 3 a donc dans le cas particulier d'un appareil de tomographie, une forme cylindrique de diamètre inférieur à celui du cylindre formé par l'ensemble des détecteurs, mais de longueur légèrement supérieure à cet ensemble ; le conteneur est centré dans la zone de détection formée par les détecteurs et fixe par rapport à celle-ci.

Par ailleurs, dans le cas d'un conteneur torique de forme cylindrique, la membrane élastique 13 est conique et les deux chambres 13l et 13g sont toriques, la chambre 13l entourant de préférence la chambre 13g.

Ce conteneur 3 est réalisé dans un matériau transparent aux rayonnements ionisants, ce matériau étant par exemple de l'aluminium.

Les deux enceintes 15l et 15g du magasin 5 sont constituées par exemple par deux soucoupes réunies suivant leur plus grande section par la membrane élastique 15. Le magasin de stockage comprend des moyens pour arrêter les rayonnements ionisants constitués par les parois mêmes du magasin 5 ou par des protections telles que des plaques 7 en plomb entourant le magasin 5. Il en est de même de la canalisation 9.

Les moyens 21 d'introduction ou d'évacuation de gaz comprennent, par exemple, un ballon 21 contenant du gaz sous pression relié à la chambre 13g par une canalisation 21b. Ce ballon 21a est ouvert sur la chambre 13g.

Par ailleurs, un manomètre 21c peut être relié à ce ballon 21a de gaz pour surveiller les différents transferts de la source 1 ou la rupture éventuelle de la membrane 13.

Les moyens 23, 25 d'introduction et d'évacuation de gaz sont séparés en des moyens 23 d'introduction de gaz et des moyens 25 d'évacuation de gaz.

Les moyens 23 d'introduction de gaz comprennent par exemple des moyens de stockage de gaz tels qu'un réservoir 23a relié à l'enceinte 15g par une canalisation 23c. Cette canalisation 23c comporte un détendeur de gaz 23d et des moyens d'ouverture et de fermeture tels qu'une électrovanne 23b.

Les moyens 25 d'évacuation de gaz comprennent par exemple un vase 25a relié à l'enceinte 15g par l'intermédiaire d'une canalisation 25c reliée à la canalisation 23c. Ce vase est relié en outre à une canalisation 25d ouvert à l'air ambiant. La canalisation 25c comprend des moyens d'ouverture et de fermeture tels qu'une électrovanne 25b et un limitateur de pression tel qu'un ressort taré 25e placé dans une canalisation 25f en parallèle de l'électrovanne 25b, les extrémités de la canalisation 25f étant reliées à la canalisation 25c. Le vase 25a permet en cas de rupture de la membrane 15 de recueillir la source radioactive liquide 1.

Un patient 30 est placé au centre du conteneur 3 pour être examiné, uniquement lorsque le conteneur 3 ne contient plus la source 1.

Le reste de la description permet de comprendre le fonctionnement du dispositif d'étalonnage de l'invention.

Lorsque l'électrovanne 23b est fermée et l'électrovanne 25b est ouverte, l'enceinte 15g est à pression atmosphérique. La source liquide 1 contenue dans la chambre 13l est transférée dans l'enceinte 15l sous l'action du gaz sous pression provenant du ballon 21a qui repousse la membrane 13 contre la paroi de la chambre 13l. Ce gaz se détend dans la chambre 13g qui augmente de volume tandis que le volume de la chambre 13l diminue. Par ailleurs, la membrane 15 est repoussée contre la paroi de l'enceinte 15g par la source 1 ; le volume de l'enceinte 15g diminue donc tandis que le volume de l'enceinte 15l augmente.

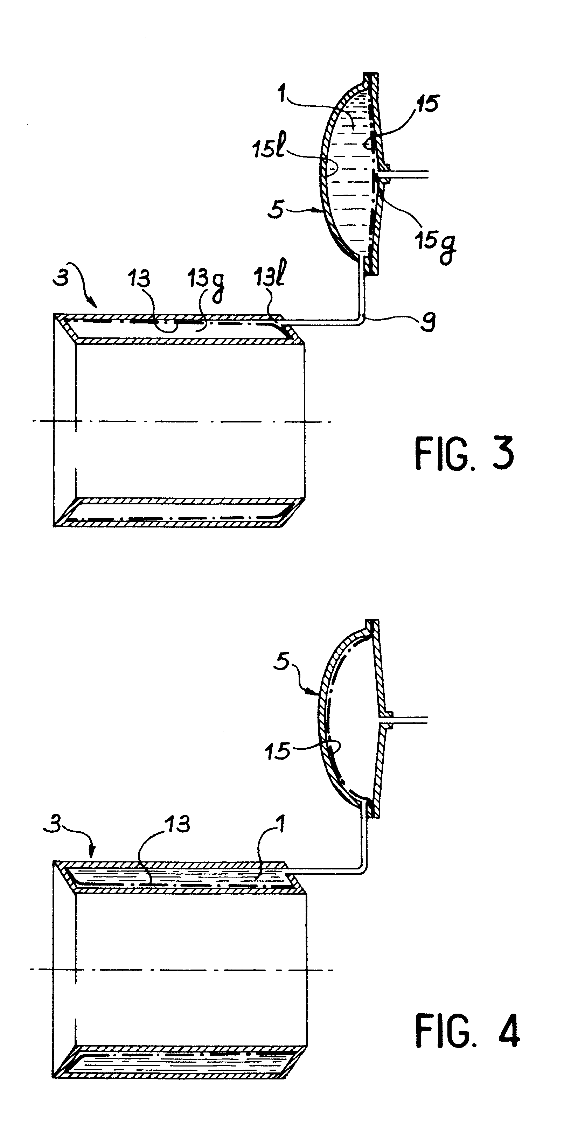

Ainsi que représenté figure 3, lorsque la totalité de la source 1 a été transférée dans l'enceinte 15l, le volume de l'enceinte 15l et le volume de la chambre 13g sont maximum et tendent à être égaux respectivement au volume total du magasin 5 et au volume total du conteneur 3. Les volumes de l'enceinte 15g et de la chambre 13l sont donc pratiquement nuls.

Pour transférer la source radioactive liquide 1 de l'enceinte 15l à la chambre 13l, on ferme l'électrovanne 25b et on ouvre l'électrovanne 23b. Du gaz contenu dans le réservoir 23a, détendu par le détendeur 23d, pénètre dans la chambre 15g par la canalisation 23c. Ce gaz repousse la membrane 15 contre la paroi de l'enceinte 15l. La source liquide 1 est donc chassée de l'enceinte 15l au profit de la chambre 13l. La membrane 13 est de ce fait repoussée contre la paroi de la chambre 13g, le gaz auparavant dans la chambre 13g étant comprimé dans le ballon 21a.

Lorsque la totalité de la source 1 est contenue dans la chambre 13l, comme représenté figure 4, le volume de la chambre 13l et le volume de l'enceinte 15g sont maximum et sont pratiquement équivalents respectivement au volume total du conteneur 3 et au volume total du magasin 5. Les volumes de la chambre 13g et de l'enceinte 15l sont alors pratiquement nuls.

La figure 2 représente une phase intermédiaire de celles représentées respectivement sur la figure 3 et la figure 4. Cette phase intermédiaire correspond à une phase de transfert de la source 1 de la chambre 13l à l'enceinte 15l ou inversement.

L'utilisation de membranes élastiques dans le dispositif de l'invention permet de s'affranchir des bulles de gaz inhérentes aux sources radioactives liquides. Cette homogénéité de la source 1 est également dûe à la forme du conteneur et à la présence de la membrane. Aussi, aucun mouvement supplémentaire de la source 1 n'est nécessaire pour s'affranchir de ces inhomogénéités.

Par ailleurs, les membranes élastiques, la forme du conteneur et la forme du magasin permettent d'obtenir respectivement un volume résiduel minimal dans le conteneur et dans le magasin. Ces volumes résiduels minimals permettent de transférer pratiquement la totalité de la source radioactive liquide de la chambre 13l à l'enceinte 15l ou inversement. Un volume résiduel correspond au volume restant entre une paroi du conteneur ou du magasin et la membrane correspondante lorsque celle-ci est repoussée contre ladite paroi.

L'exemple de réalisation décrit précédemment n'est pas limitatif, d'autres formes de conteneur et de magasin peuvent être envisagées sans pour autant sortir du cadre de l'invention. La forme du conteneur varie en particulier en fonction des appareils de médecine nucléaire pour lesquels il est utilisé, sa forme s'adaptant à la zone de détection de ces appareils.

## Revendications

1. Dispositif d'étalonnage d'un appareil de médecine nucléaire servant à l'observation d'un patient préalablement rendu radioactif, comprenant une source radioactive liquide (1) servant, en dehors de la présence d'un patient (30), à l'étalonnage des détecteurs de rayonnements ionisants de l'appareil, logée dans un conteneur (3) pouvant contenir ladite source, caractérisé en ce qu'il comprend un magasin de stockage (5) communiquant avec le conteneur par au moins une canalisation (9), des moyens pour transférer la source radioactive liquide dudit magasin de stockage au conteneur et inversement, le conteneur étant fixe par rapport audit appareil, le magasin de stockage ayant un volume au moins égal à celui du conteneur, et des moyens (7) pour arrêter les rayonnements ionisants.

2. Dispositif d'étalonnage selon la revendication 1, caractérisé en ce que les moyens pour transférer la source radioactive liquide (1) comprennent une première membrane élastique (13) étanche, disposée dans le conteneur (3) de façon à créer dans ledit conteneur une première et une deuxième chambres (13l, 13g) à volumes variables, la première chambre (13l) étant reliée au magasin de stockage (5) et la deuxième chambre (13g) étant reliée à des premiers moyens (21) d'introduction et d'évacuation de gaz sous pression.

3. Dispositif d'étalonnage selon l'une quelconque des revendications 1 et 2 caractérisé en ce que les moyens pour transférer la source radioactive liquide (1) comprennent une deuxième membrane élastique (15) étanche, disposée dans le magasin de stockage (5) de façon à créer dans ledit magasin une première et une deuxième enceintes (15l, 15g) à volumes variables, ladite première enceinte (15l) étant reliée au conteneur (3) et la deuxième enceinte (15g) étant reliée à des deuxièmes moyens (23, 25) d'introduction et d'évacuation de gaz sous pression.

4. Dispositif d'étalonnage selon l'une quelconque des revendications 2 et 3, caractérisé en ce que le conteneur (3) est torique et la première membrane (13) est conique, les première et deuxième chambres (13l, 13g) étant toriques, l'une (13l) desdites chambres entourant l'autre (13g).

5. Dispositif d'étalonnage selon l'une quelconque des revendications 3 et 4, caractérisé en ce que les première et deuxième enceintes (15l, 15g) ont respectivement une forme de soucoupe et sont réunies suivant leur plus grande section par la deuxième membrane (15).

6. Dispositif d'étalonnage selon l'une quelconque des revendications 2 à 5, caractérisé en ce que les premiers moyens (21) d'introduction et d'évacuation de gaz comprennent un ballon (21a) de réserve de gaz sous pression relié par une canalisation (21b) à la deuxième chambre (13g).

7. Dispositif d'étalonnage selon l'une quelconque des revendications 3 à 6, caractérisé en ce que les deuxièmes moyens (23, 25) d'introduction et d'évacuation de gaz comprennent :
- des moyens (23) d'introduction de gaz comprenant des moyens (23g) de stockage de gaz reliés à la deuxième enceinte (15g) par une première canalisation (23c) comportant des premiers moyens (23b) d'ouverture et de fermeture.
- des moyens (25) d'évacuation de gaz par une mise à l'air ambiant de la deuxième enceinte (15g), lesdits moyens comportant des deuxièmes moyens (25b) d'ouverture et de fermeture.

8. Dispositif d'étalonnage selon la revendication 7, caractérisé en ce que les moyens (25) d'évacuation de gaz par une mise à l'air ambiant de la deuxième enceinte (15g) comprennent un vase (25a) relié à la première canalisation (23c) par une deuxième canalisation (25c), ce vase étant en outre relié à une troisième canalisation (25d) ouverte à l'air ambiant, ladite deuxième canalisation comprenant les deuxièmes moyens d'ouverture et de fermeture et des moyens (25e) de limitation de pression.

9. Dispositif d'étalonnage selon l'une quelconque des revendications 2 à 8, caractérisé en ce qu'il comprend en outre des moyens (27) d'évacuation d'air de la première enceinte (15l) et de la première chambre (13l), lesdits moyens étant reliés à la première enceinte (15l) et/ou à la première chambre (13l).

10. Dispositif d'étalonnage selon l'une quelconque des revendications 2 à 9, caractérisé en ce qu'il comprend aussi des moyens (29) pour introduire et évacuer la source radioactive liquide (1), lesdits moyens étant reliés à la première chambre (13l) et/ou à la première enceinte (15l).

11. Procédé de mise en oeuvre du dispositif d'étalonnage selon l'une quelconque des revendications 1 à 10, caractérisé en ce que la source radioactive liquide (1) étant dans la première enceinte (15l), on transfère la source radioactive liquide dans la première chambre (13l) en introduisant du gaz sous pression dans la deuxième enceinte (15g) par les deuxièmes moyens (23, 25) d'introduction et d'évacuation de gaz sous pression.

12. Procédé de mise en oeuvre du dispositif d'étalonnage selon l'une quelconque des revendications 1 à 11, caractérisé en ce que la source radioac tive liquide (1) étant dans la première chambre (13l), on transfère la source radioactive liquide dans la première enceinte (15l) en évacuant le gaz de la deuxième enceinte (15g) par les deuxièmes moyens (23, 25) d'introduction et d'évacuation de gaz sous pression.

0228309

FIG. 1

0228309

# FIG. 2

0228309

FIG. 3

FIG. 4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | PHYSICS IN MEDICINE & BIOLOGY, vol. 24, no. 1, janvier 1979, pages 188-192, The Institute of Physics, GB; K.J. MURRAY et al.: "A new phantom for the assessment of nuclear medicine imaging equipment" * Page 189, point 2 "Description"; figure 1 * | 1 | G 01 T    1/29 G 01 T    1/161 |
| A | US-A-3 917 124  (D.E. KIFER) * Résumé; figures * | 1,3,5 | |
| A | DE-A-2 535 182  (PETERS & WESTEBBE KG) * Figure 1 * | 1 | |

DOMAINES TECHNIQUES RECHERCHES (Int. Cl.4)

G 01 T
G 21 K
B 67 D

Le présent rapport de recherche a été établi pour toutes les revendications

| Lieu de la recherche | Date d'achèvement de la recherche | Examinateur |
|---|---|---|
| LA HAYE | 06-02-1987 | DATTA S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82